# EUROPEAN PATENT APPLICATION

(11) **EP 1 267 167 A1**
(43) Date of publication of application: **18.12.2002**
(21) Application number: 02253847.4
(22) Date of filing: 31.05.2002
(51) Int. Cl.: G01N 33/50, G01N 33/68, A61K 38/16, A61K 39/00, A61K 39/395, A61K 39/42

(54) **Method for identifying HCV entry factors**

(30) Priority: 15.06.2001 GB 0114629
(71) Applicant: Pfizer Limited, Sandwich, Kent CT13 9NJ (GB); Pfizer Inc., New York, New York 10017 (US)
(72) Inventor: Jenkins, Timothy Mark, Pfizer Global R & D, Sandwich, Kent CT13 9NJ (GB); Lavender, Helen Bostock, Pfizer Global R & D, Sandwich, Kent CT13 9NJ (GB); Perros, Manoussos, Pfizer Global R & D, Sandwich, Kent CT13 9NJ (GB)
(74) Representative: Hayles, James Richard

(57) **Abstract**

The invention relates to methods for identifying and assaying candidate HCV entry factor(s), methods for identifying and assaying candidate modulators of HCV entry, methods for identifying permissive cell lines and methods for identifying cellular factors affecting entry/fusion, as well as to the modulators/factors themselves.

## Description

### Field of the Invention

The invention relates to Hepatitis C Virus (HCV) entry factors, modulators of HCV entry and to methods for identifying and assaying them.

### Background to the Invention

The presence and spread of hepatitis C virus (HCV) in human populations is a major health concern. HCV has a world-wide distribution affecting up to 3% of the world's population. The prevalence of hepatitis C virus infection in the US is approximately 1.8%.

Chronic hepatitis C is the leading cause of decompensated liver disease requiring liver transplantation, a major cause of hepatocellular carcinoma (HCC), and has been implicated in the development of cirrhosis of the liver, overt B-cell non-Hodgkin's lymphoma and idiopathic pulmonary fibrosis. Recurrent hepatitis C infection and subsequent graft failure are increasingly recognised problems following orthotopic liver transplantation.

There is a need for systems/methods which facilitate the study of HCV.

HCV replicates inside its host cells. HCV particles enter the host cells by a poorly understood mechanism. There is a need for new ways in which viral entry could be studied in order to develop better ways of understanding and/or combatting the disease. There is a need for methods/techniques for the identification of viral entry factors, and for modulators and/or inhibitors of viral entry such as HCV entry.

The present invention seeks to address problem(s) associated with the prior art.

### Summary of the Invention

Entry of viruses such as enveloped viruses into their eukaryotic host cells involves fusion of the viral lipid envelope with membrane(s) of the host cell. For some species of virus, entry may be promoted or facilitated by viral envelope proteins. For some species of virus, this fusion step may be reproduced *in vitro* by expressing viral envelope protein(s) on the surface of mammalian cells. These cells may then be induced to fuse with susceptible cells, for example by layering the cells in culture.

In a broad aspect, the present invention relates to the viral entry of HCV.

In another broad aspect, the present invention concerns a syncytium forming assay to identify potential anti-HCV agents.

A syncytium is a cell which is the product of membrane fusion between two or more cells. Where said cells have nuclei, the resulting syncytium will be multinucleate. For some viral species, syncytium formation may be observed for example when viral envelope proteins are expressed on the surface of a mammalian cell and cell fusion is induced. A system which can reproduce the syncytium forming fusogenic potential of virus envelope glycoproteins is a valuable tool in the study of that virus. In a broad aspect, the present invention provides systems for the study of HCV entry/fusion.

Cell surface expression of HCV envelope proteins has been reported in the literature. However, prior attempts to establish syncytium forming systems have been unsuccessful (e.g. Takikawa *et al,* (2000) *J. Virol.* vol 74, pp 5066-5074; Flint *et al,* (1999) *J. Virol.* vol 73 pp 6782-6790). It is disclosed herein for the first time that HCV envelope glycoproteins such as E1 and E2 are capable of supporting syncytium formation. In a broad aspect, the present invention relates to syncytium formation.

A system for syncytium formation mediated by HCV proteins is not present in the prior art, where attempts to produce such a system have foundered. The biology of HCV infection suggests that making such a system may not be possible.

It is surprisingly disclosed herein that a system for syncytium formation mediated by HCV proteins can be produced. The invention is based on the discovery of certain conditions which reproducibly promote syncytium formation mediated by HCV proteins.

Thus, the invention provides, *inter alia,* methods for identifying candidate HCV entry factor(s), methods for identifying and assaying candidate modulators of HCV entry, methods for identifying permissive cell lines and methods for identifying cellular factors affecting entry/fusion. Aspects of the invention are described below.

The methods of the present invention typically involve a syncytium formation assay comprising mixing test cell(s) and target cell(s), placing them into near proximity and/or contact with one another, incubating the resulting cell mixture, allowing sufficient time for syncytium formation to take place, and assaying the syncytium formation. Particular conditions, illustrative examples and variations of the methods are described herein.

The syncytium formation is influenced by determinants on the cell surface. In the assays of the invention, the cell surface of the test cell comprises factor(s) capable of supporting syncytium formation. Typically these factor(s) include molecules found on the virus surface, such as on the viral envelope of HCV, for example E1 and/or E2 glycoproteins. In one embodiment, a target cell is tested for its ability to support syncytium formation with the test cell(s). This syncytium formation reflects the fusion of viral envelope with cell membrane at the time of viral entry. Syncytium formation is thus used herein as a direct biological model for viral entry.

In a first embodiment, the invention is used to identify HCV entry factor(s) by placing the factor(s) on the surface of a target cell and determining whether the factor(s) are capable of supporting syncytium formation i.e. viral entry.

Thus, in one aspect, the invention relates to a method for identifying a candidate HCV entry factor comprising expressing a target polypeptide on the surface of a target cell, providing a test cell, mixing said test and target cells, incubating the cell mixture, and assaying for syncytium formation, wherein the detection of syncytium formation indicates that the target polypeptide is a candidate HCV entry factor.

The target polypeptide may be any polypeptide of interest, for example derived from expression libraries, cell surface molecules, translated viral open reading frames (ORFs), or any other molecule of interest. The term 'polypeptide' as used herein embraces polypeptides and mature proteins, including post-translationally modified proteins, for example glycoproteins.

Fusogenic polypeptides, i.e. polypeptides which support and/or promote syncytium formation, are candidate HCV entry factors according to the present invention. The potency (e.g. fusogenicity) of the candidate entry factor(s) may be estimated by expressing the factor(s) at different levels, performing the assay as described, and looking for a relationship between the expression level of the factor(s) and the extent of syncytium formation. As a general guide, the greater the level of syncytium formation, the more potent the factor. If a negative relationship is found (i.e. the more factor present, the less syncytium formation observed), the factor may be a virus entry inhibitor. If a positive relationship is found (i.e. the greater the amount of factor expressed, the greater the amount of syncytium formation observed), the factor may be a virus entry stimulating factor or a virus entry receptor. Thus, in another aspect, the present invention provides a method for identification of virus entry inhibitors and/or virus entry stimulating factors and/or virus entry receptors.

In the methods of the present invention described above and below, the test cell is preferably a eukaryotic cell, more preferably a mammalian cell, most preferably a Chinese Hamster Ovary (CHO) derived cell. Thus, in another aspect, the invention relates to methods as described herein wherein the test cell is a Chinese Hamster Ovary (CHO) derived cell.

In the methods of the present invention described above and below, the test cell preferably has certain characteristics. Cell surface expression of Hepatitis C Virus envelope glycoproteins on the test cell drives syncytium formation with permissive cells under the conditions of the assay(s) as set out herein. These conditions are different from prior art assay conditions and are discussed more fully below.

Preferably the test cell expresses one or more Hepatitis C Virus (HCV) proteins such as envelope proteins. Thus, in another aspect, the invention relates to methods as described herein wherein the test cell comprises one or more Hepatitis C Virus (HCV) envelope protein(s). Preferably the HCV envelope proteins are expressed on the cell surface.

Preferably the envelope protein(s) comprise HCV E1 and/or E2, more preferably the envelope protein(s) comprise E1 and E2. Thus, in another aspect, the invention relates to methods as described herein wherein the test cell comprises Hepatitis C Virus (HCV) E1 and E2 envelope proteins. These proteins may be present as discrete proteins, or may be comprised in fusion proteins. Substantially all of E1/E2 may be present, or part(s) thereof may be present on the surface of the test cell. Most preferably the HCV envelope protein parts present on the test cell are as described in Takikawa *et al.* (Takikawa *et al* (2000) *J. Virol.* vol 74 pp 5066-5074).

'Mixing' of the cells describes bringing the test and target cells into close proximity and/or contact with one another. This may be accomplished by any means known in the art such as layering, where one population of cells (e.g. test cells) is overlaid with the second (e.g. target cells). Preferably the cells are mixed in suspension, e.g. two suspensions of cells (one of target cells and one of test cells) are placed into the same vessel so that the cells may mix. Thus, in another aspect, the invention relates to methods as described herein wherein the test cells and target cells are mixed in suspension.

The conditions for incubation may be varied according to the preferences of the cells being used as is well known in the art of tissue culture/propagation. When adherent cell lines are used, the cells are preferably given the opportunity to adhere to a solid substrate. Such substrates are well known in the art and include for example tissue culture plastic e.g. flasks, well dishes or similar containers manufactured for use with eukaryotic (mammalian) cell culture. Thus, in another aspect, the invention relates to methods as described herein wherein the cells are incubated in the presence of a solid tissue culture substrate. Highly preferred incubation conditions are incubation at 37° C, 5% CO₂ in culture medium B (see below for details) over tissue culture treated plastic. However, the method can also be used with either one or both cell lines in suspension.

Syncytia formation will still occur when cells are in suspension, provided that the cell concentration is sufficiently high for the cells to get into contact with each other. For cell suspensions, any culture vessel could be used; preferably plastic dishes or multiwell plates, even more preferably dishes or multiwell plates for cell cultures.

The term 'seeding' may be used to describe the act of placing the cells into the medium/vessel for incubation.

Preferred times for incubation are for 4-36 hours or even more, preferably for 4-36 hours, preferably for 4-30 hours, more preferably for 16-30 hours, even more preferably for 16-24 hours, most preferably for about 20 hours. Syncytium formation (when occurring) may be observed from about 20 hours. Thus, in another aspect, the invention relates to methods as described herein wherein the cells are incubated for at least about 20 hours.

Confluency describes the presence of a continuous cell layer. As used herein, the term embraces confluent as well as near-confluent or substantially confluent cell layer(s). Near-confluent or substantially confluent cell monolayers comprise a majority of cells in sufficient proximity that the layer may be regarded as essentially confluent, i.e. with only a minority of cells which are not in contact with their neighbours. The cell density giving confluency may be empirically determined taking into account cell volume, cell shape/spread and the surface area of the available substrate (e.g. the tissue culture plastic). Confluency may be easily examined/confirmed microscopically as is well known in the art. Confluency or substantial confluency may be obtained for example by incubating 6 well plates such as Nunc 6 well plates (Catalogue number 152795) with 200 x 10⁴ cells/well, or incubating 96 well plates such as Costar 96 well plates (Catalogue number 3598) with 8 x 10⁴ cells/well. Thus, in another aspect, the invention relates to methods as described herein wherein cells are incubated at a density giving substantial confluency. Preferably cells are incubated at a density giving full confluency. Superconfluency occurs when cells are even more densely arranged than a confluent monolayer (see examples).

Preferably, test cells are present at a lower concentration than target cells. Thus, in another aspect, the invention relates to methods as described herein wherein test cells are present at a lower concentration than target cells. Preferably the test cell to target cell ratio is in the range 1:2 to 1:20, more preferably in the range of 1:2 to 1:10, even more preferably in the range of 1:2 to 1:8. Thus, in another aspect, the invention relates to methods as described herein wherein the test cell to target cell ratio is in the range 1:2 to 1:8.

For specific test and target cell combinations, however, it may be advantageous to use test cells at a higher concentration than the target cells. Thus, in another aspect, the invention relates to methods as described herein wherein test cells are present at a higher concentration than target cells. Preferably, the test cell to target cell ratio is in the range of 20:1 to 2:1, more preferably in the range of 10:1 to 2:1, even more preferably in the range of 8:1 to 2:1. Thus, in another aspect, the invention relates to methods as described herein wherein the test cell to target cell ratio is in the range 2:1 to 8:1.

In another aspect, the invention relates to a method for determining whether cell(s) are permissive or non-permissive for syncytium formation. Thus the invention provides a method for identifying a permissive cell comprising providing a test cell, providing a candidate cell, mixing said test and candidate cells, incubating the cell mixture, and assaying for syncytium formation, wherein the detection of syncytium formation indicates that the candidate cell is a permissive cell. A permissive cell is a cell able to be fused with another cell such as a test cell *via* the action of HCV factor(s) such as polypeptide(s) such as glycoproteins. A permissive cell is thus one which may be susceptible to HCV entry/infection. The cell may be from an established cell line, or may be a primary cell or any other cell of interest. Preferably the cell is from a cell line.

According to the present invention, syncytium formation forms a biological model for viral entry such as HCV entry. Therefore, by conducting syncytium formation assays of the present invention in the presence and absence of one or more test molecule(s), the effects of the test molecule(s) on syncytium formation (and therefore the effects on HCV entry) may be assayed. Such test molecules are thereby assayed for their properties as modulators of HCV entry. Thus, in another aspect, the invention relates to a method for identifying a candidate modulator of HCV entry comprising providing test cells, providing target cells, mixing said test and target cells, dividing the mixture into two samples, introducing a test molecule into the first sample, incubating the first and second samples and assaying the samples for syncytium formation wherein a difference in the level of syncytium formation between the first and second samples indicates that the test molecule is a candidate modulator of HCV entry. It is envisaged that the cell mixture could be divided into a minimum of two samples. The cell mixture may be advantageously divided into at least two samples e.g. 2, 3, 4, 6, 10, 12, 20, 24, 30, 48, 96, 384 or even more samples, for example it may be advantageous to carry out method(s) of the present invention in multi-chamber device(s) such as a 96-well plate(s) or similar device(s), when the cell mixture would be split into as many or as few samples as are required for the particular application to which the method is being put. Such choices may be routinely made by a person skilled in the art. Thus, in another aspect, the invention relates to a method for identifying a candidate modulator of HCV entry comprising providing test cells, providing target cells, mixing said test and target cells, dividing the mixture into a first and at least one further sample(s), introducing a test molecule into at least one further sample, incubating the first and further sample(s) and assaying the samples for syncytium formation wherein a difference in the level of syncytium formation between the first and further sample(s) indicates that the test molecule is a candidate modulator of HCV entry.

The choice of target cell may be advantageously varied in order to facilitate the assay of an inhibitory and/or a stimulatory modulator of HCV entry. For example, if the target cell is permissive, then the identification/assay of modulators having inhibitory effect(s) on HCV entry is facilitated. Alternatively, if the target cell is non-permissive, then the identification/assay of modulators having stimulatory effect(s) on HCV entry is facilitated. Such stimulatory modulators may be receptors for the virus and/or docking proteins capable of facilitating viral entry into the cell. Thus, in another aspect, the present invention provides a method for identification of virus entry inhibitors and/or virus entry receptors and/or facilitating factors.

It will be apparent to a person skilled in the art that this method may be easily adapted in order to estimate the particular efficacy of a molecule of interest. For example, if the method is performed in parallel with different concentrations of the test molecule, and the level of syncytium formation are compared between the different treatments, then the particular activity of the modulator may be assayed. Clearly, this technique may be advantageously applied to the comparison of different formulations, salt forms, combinations or similar variants of molecules of interest for their varying effects on HCV entry (syncytium formation) according to the present invention.

A molecule of interest may be any chemical entity which it is desired to test. The molecule may be a small chemical compound, or may be a polymeric molecule such as a polypeptide, protein, glycoprotein or similar molecule. The molecule may be or may be derived from a viral envelope glycoprotein. Thus, in another aspect, the invention relates to a system for identifying and/or assaying the syncytium forming potential of a candidate protein such as a Hepatitis C Virus envelope glycoprotein.

Thus, in another aspect, the invention relates to a modulator of fusion identified by a method as described herein.

The dependency of syncytium formation on E2 expression is demonstrated herein, for example by competition studies with recombinant E2 protein. E2 protein is a modulator of syncytium formation according to the present invention. Thus, in another aspect, the invention relates to the use of E2 as a modulator of HCV entry. It is demonstrated that HCV fusion may be inhibited by E2. Thus, in another aspect, the invention relates to the use of E2 in the manufacture of a medicament for treatment of HCV infection. The treatment of HCV infection may comprise curative and/or prophylactic treatment.

In another aspect, the invention relates to the use of E1 as a modulator of HCV entry. In another aspect, the invention relates to the use of E1 in the manufacture of a medicament for treatment of HCV infection.

In another aspect, the invention relates to the use of an antibody to E1 or an antibody to E2 as a modulator of HCV entry.

In another aspect, the invention relates to the use of an antibody to E1 or an antibody to E2 in the manufacture of a medicament for treatment of HCV infection.

It is demonstrated herein that an antibody to LDL receptor is a modulator of syncytium formation according to the present invention. Thus, in another aspect, the invention relates to the use of anti-LDL receptor antibody as a modulator of HCV entry. It is demonstrated that HCV fusion may be inhibited by anti-LDL receptor antibody. Thus, in another aspect, the invention relates to the use of anti-LDL receptor antibody in the manufacture of a medicament for treatment of HCV infection. The treatment of HCV infection may comprise curative and/or prophylactic treatment.

In another aspect, the invention relates to the use of an antibody to an HCV entry factor identified by the methods described herein as a modulator of HCV entry. In another aspect, the invention relates to the use of an antibody to an HCV entry factor identified by the methods described herein in the manufacture of a medicament for treatment of HCV infection.

Underlying syncytium formation is cell-cell fusion. Syncytia may be formed by many cells simultaneously fusing with one another, or may be formed serially by one cell fusing with another cell, forming a small binucleate syncytium which then fuses with one or more further cells, thereby enlarging the syncytium. In practice, large syncytia are formed by a mixture of simultaneous and serial fusions. Clearly, observation of syncytia is a direct indicator of cell fusion taking place. Thus, in another aspect, the present invention relates to a method for monitoring cell fusion, said method comprising providing a test cell, providing a target cell, mixing said test and target cells incubating the cell mixture, assaying for syncytium formation and inferring the level of cell fusion from the level of syncytium formation.

The syncytium formation assays/methods of the present invention reflect real biological events (i.e. syncytium formation) as distinct from those of a "fusion" assay report (Takikawa *et al.* (2000) *J*. *Virol.* vol 74 pp 5066-5074) which relied on indirect reporters in the absence of syncytium formation. No syncytium formation is observed by Takikawa *et al.* using for example low power light microscopy, whereas in the present invention, extensive syncytium formation is observed via low power light microscopy.

### Detailed Description of the Invention

### Permissive and Non-Permissive Cell Lines

Permissive cell line(s) according to the present invention are cell line(s) which are capable of fusing with test cells, forming syncytia as discussed herein.

Permissive cells preferably form syncytia under at least the conditions set out in the Examples. Permissive cells preferably form syncytia under the conditions of the Examples with at least the CHO E1E2 test cells discussed therein.

Permissiveness can be determined according to the present invention, for example as set out in Examples 1 and/or 2.

Permissive cell lines useful in the methods and/or assays of the present invention include HepG2 (human hepatoma) and Huh7 (human hepatoma).

The term 'permissive cell line' as used herein refers to a cell line found to be permissive, for example under the conditions described above. The permissiveness of such cell line(s) may be advantageously modified or modulated by contacting such cell(s) with factor(s) capable of temporarily or permanently altering the permissiveness of said cell line. This is an advantageous feature of the present invention, but does not affect the definition of permissive cell line as set out above.

Permissive cell lines may be rendered temporarily non-permissive, for example by contacting said cell line(s) with fusion inhibitor(s), or with inhibitor(s) of viral entry. Non-permissive cell lines according to the present invention are cell line(s) which do not form syncytia under the conditions set out in Examples 1 and/or 2.

Cell line(s) believed to be non-permissive include Chang, Hela (human cervical carcinoma), Hek293 (Human Embryonic Kidney), Cos-7 (African Green Monkey kidney cells, SV40-transformed) and CHO (Chinese Hamster Ovary) cells.

A person skilled in the art will realise that a cell line which appears to be non-permissive according to the tests outlined above may be rendered permissive by variation of or contact with one or more factor(s). For example, a non-permissive cell line may be rendered permissive by expression of entry receptor(s) on its cell surface, and/or by expression of entry factors within the cells. A non-permissive cell line may be found to be permissive for one or more type(s) or strain(s) of HCV other than the particular HCV from which the E1E2 proteins as used in the test outlined above were derived. A non-permissive cell line might be rendered permissive by contacting it with one or more factor(s) capable of promoting fusion, or capable of enhancing, augmenting or otherwise modulating virus entry. Examples of factor(s) affecting fusion/permissiveness are discussed herein.

Factors affecting fusion/permissiveness may be cellular factors. Such factors may have a genetic basis in the cell in question. It will be apparent to a person skilled in the art that the provision of an assay for permissive/non-permissive cell lines as described herein facilitates the dissection of the basis of cellular factor(s) affecting permissiveness. For example, it is possible to employ subtractive cloning to isolate mRNAs which are unique to permissive cells or which differ in expression levels between permissive and non-permissive cells. Differential display or microarray techniques may advantageously be used to investigate differences between permissive and non-permissive cells. Factor(s) affecting permissiveness identified according to the present invention are candidate HCV entry factor(s).

The provision of an assay for permissiveness as described herein also makes possible the cloning of factor(s) affecting permissiveness. For example, a cDNA expression library or gene of interest may be introduced into a population of non-permissive cells, said population may be screened for permissiveness and cDNA(s) cloned which confer permissiveness onto the (previously non-permissive) host cell. Similarly, a permissive cell population may be transfected with a cDNA library or gene of interest, and gene(s) which have an inhibitory effect on fusion/HCV entry could be cloned by their ability to make a previously permissive cell non-permissive.

### Test Cell

The term 'test cell' as used herein relates to a cell(s) with which target cell(s) will be mixed in the course of an assay. The test cell is permissive, and as is disclosed herein possesses feature(s) which allow syncytium formation/fusion with a permissive target cell to take place. Preferably, the test cell is a mammalian cell expressing polypeptide(s) derived from one or more HCV envelope protein(s) on its cell surface. A preferred test cell according to the present invention is a Chinese Hamster Ovary (CHO) cell expressing HCV-derived E1 and E2 polypeptides, which cell line is abbreviated to 'CHO E1E2' herein. In the Examples, the E1/E2 are derived from HCV type 1b, but E1/E2 from other genotypes of HCV should be considered to be embraced by the term.

### Target Cell

The term 'target cell' as used herein refers to the cell(s) which are being tested for their permissiveness for syncytium formation/cell fusion. The target cell(s) may be tested for any measurable aspect of syncytium formation and/or fusion. For example, it may be desirable to use a target cell in combination with a test cell, which pair of cells are known to fuse efficiently in the course of assay, and to use this system to identify and/or to further characterise/investigate candidate modulator(s) of fusion/viral entry.

### E1/E2

As used herein, the terms 'E1' and 'E2' refer to molecules such as polypeptides comprising some or all of the HCV E1 and E2 proteins respectively. These proteins may be present as discrete proteins, or may be comprised in fusion proteins. Substantially all of E1/E2 may be present, or part(s) thereof may be present. For example, when E1/E2 is expressed on the surface of the test cell, E1 and E2 preferably comprise soluble domain(s) or extracellular/ectodomain(s) of the E1 and E2 proteins respectively, or at least part(s) thereof. Parts of the E1/E2 molecule(s) may be substituted, for example the transmembrane domain(s) of the HCV E1/E2 proteins may be partly or wholly truncated and optionally replaced with transmembrane domain(s) from other sources(s) such as from VSV-G and/or from Sindbis virus. Preferably the HCV envelope protein parts present on the test cell are as described in Takikawa *et al.* (Takikawa *et al.* (2000) *J.Virol.* vol 74 pp 5066-5074). In the Examples, the E1/E2 are derived from HCV type 1b, but E1/E2 from other genotypes of HCV should be considered to be embraced by the term.

### Syncytium Formation

A syncytium is a multinucleate cell which is the product of membrane fusion between two or more cells.

The term 'syncytium' preferably has its normal meaning in the art. Preferably the meaning is consistent with the use of the term in the art, such as in Flint *et al.* (1999) *J.Virol.* vol 73 pp 6782-6790. Preferably the meaning is consistent with use of the term in Takikawa *et al.* (2000) *J. Virol.* vol 74 pp 5066-5074.

As used herein, the term 'syncytium formation' preferably refers to cell fusion event(s) which involve more than two cells. Syncytia according to the present invention preferably comprise three or more nuclei, preferably four or more nuclei, preferably more than six nuclei, preferably ten or more nuclei, preferably 20-50 nuclei, or even more. Highly preferred syncytia are characterised by attaining a size sufficiently large to be detected by low power light microscopy. Most highly preferred syncytia formed according to the present invention are characterised by detachment from the substrate to which the unfused cells giving rise to the syncytia were adhered. Preferably this detachment can be assessed visually (unaided) or with recourse to only low-power microscopy. Preferably syncytia according to the present invention are formed within about 20 hours from cell seeding.

In a preferred embodiment of the present invention, significant syncytium formation is characterised by fusion of about 20% of cells in the mixture, preferably fusion of at least about 20% of cells, more preferably fusion of at least about 20-40% of cells, even more preferably fusion of at least about 20-60% of cells, even more preferably fusion of at least about 20-80% of cells, most preferably fusion of at least about 20-90% of cells, or even more. These levels of fusion are preferably observed after incubation for an appropriate period, such as 20 hours, for example when the cells are incubated in the presence of a substrate in accordance with the present invention, such as in a 96 well culture plate.

Syncytium formation in HCV-infected primary liver cells has not been observed. In assays according to the present invention, cell surface expression of E1/E2 facilitates syncytium formation. Without wishing to be bound by theory, in the context of the present disclosure, the absence of HCV E1 and E2 from the cell surface of HCV-infected liver cells may explain why syncytia have not been observed during such HCV infections. Furthermore, without wishing to be bound by theory, HCV is not thought to bud from the plasma membrane, but may bud off internal organelles such as the endoplasmic reticulum, which makes it particularly surprising that the syncytium formation assays described herein may be made to work so effectively.

A system which can reproduce the syncytium forming fusogenic potential of a virus provides means for identification of virus entry inhibitors and virus entry receptor(s). Strategies for assaying/measuring syncytium formation useful in the present invention include the use of fluorescent probes to measure membrane or cytoplasmic mixing during fusion/syncytium formation, microscopic techniques and other suitable methods which are discussed in more detail below.

It is an advantageous feature of the present invention that the cells are plated out or adhered to a solid substrate. This feature has the advantageous effect of facilitating syncytium formation. However, this is not an essential feature of the invention, and the method can also be adapted for use with one or both cell lines in suspension. This will be especially advantageous for cell lines whose preferred culture condition is suspension culture.

It is an advantageous feature of the present invention that the cells are incubated at confluent (or near or substantially confluent as explained above) densities. This feature has the advantageous effect of promoting syncytium formation. Incubation of cells at low densities is not effective for promotion of syncytium formation.

Cells useful in the present invention are preferably maintained under selective conditions, preferably under strongly selective conditions. Maintenance of cells under such selective conditions advantageously ensures retention of the construct(s) which bear the selectable markers being selected for.

### Modulators of Fusion

A modulator of fusion is any agent such as a molecule which has an effect on fusion. The effect of a modulator may be to enhance, accelerate, increase, stimulate or otherwise promote fusion, or the effect may be to retard, prevent, restrict, reduce or otherwise inhibit fusion. A modulator may affect the rate of fusion, or may affect its equilibrium, or may affect the final ration of fused to unfused cells, or may affect any combination of such factors. Preferred modulators according to the invention have the effect of inhibiting and/or reducing fusion, whether by prevention, restriction, down-regulation or any other mechanism.

A modulator (agent) molecule may be any chemical entity. A modulator molecule may be an organic molecule such as a small organic molecule, or may be a polymeric molecule such as a polypeptide or protein molecule. A modulator molecule may also comprise macromolecule(s) such as one or more antibodies.

In one embodiment, the present invention provides modulators of fusion, for example it is disclosed herein that HCV derived E2 polypeptide is a modulator of cell fusion/syncytium formation. Furthermore, it is demonstrated that HCV derived E1 polypeptide functions as a modulator of fusion according to the present invention. Other possible modulators include CD81 and its binding partner(s), as well as LDL receptors and their ligands.

Modulator(s) of fusion/syncytium formation as demonstrated in the assays or methods disclosed herein are candidate modulators of virus entry. Thus, the present invention relates to modulators of virus entry, and to their identification, testing and/or validation.

### Antibody

The term 'antibody' is to be understood broadly. The skilled person will appreciate that it includes, *inter alia,* polyclonal antibodies, monoclonal antibodies, recombinant antibodies, antibody fragments such as Fab, F(ab')₂, Fv, single-chain Fv (scFv), produced by proteolytic digestion of whole antibody molecules or recombinantly by expression of the relevant part of the antibody molecule, humanised monoclonal antibodies or fragments of monoclonal antibodies, antibodies or antibody fragments fused with other molecules such as, for example, polyethylene glycol for stabilisation.

The skilled person will be well aware of techniques to produce antibodies to a protein or glycoprotein antigen, which include traditional methods of polyclonal antibody production such as injections of antigen preparations (e.g. complete protein or glycoprotein mixed with appropriate adjuvants, or peptides derived from the sequence of the antigen coupled to a suitable carrier protein) into suitable host animals such as goats, sheep, rabbits, etc., and obtaining serum samples at appropriate time intervals. Methods for producing monoclonal antibodies are also well known to the skilled person. These include, but are not limited to, the hybridoma technique originally described by Koehler and Milstein (1975, Nature Vol 256 p495-497), the human B-cell hybridoma technique (Kosbor *et al.* (1983) Immunol Today Vol 4 p72; Cote *et al.* (1983) Proceedings of the National Academy of Sciences (USA) Vol 80 p2026-2030) and the EBV-hybridoma technique (Cole *et al.* (1985) Monoclonal Antibodies and Cancer Therapy, Alan R Liss Inc, pp. 77-96). In addition, techniques developed for the production of "chimeric antibodies", the splicing of relevant parts of mouse antibody genes to human antibody genes to obtain a molecule with appropriate antigen specificity and biological activity can be used (Morrison *et al.* (1984) Proceedings of the National Academy of Sciences (USA) Vol 81 p6851-6855; Neuberger *et al.* (1984) Nature Vol 312 p604-608; Takeda *et al.* (1985) Nature Vol 314 p452-454).

Antibodies may also be produced by inducing *in vivo* production in the lymphocyte population or by screening recombinant immunoglobulin libraries or panels of highly specific binding reagents as disclosed in Orlandi *et al.* (1989, Proceedings of the National Academy of Sciences (USA) Vol 86 p 3833-3837), and Winter G and Milstein C (1991; Nature Vol 349 p293-299).

Alternatively, Fab expression libraries may be constructed to allow rapid and easy identification of monoclonal Fab fragments with the desired specificity (Huse WD *et al.* (1989) Science Vol 256 p1275-1281).

Yet another alternative technique involves screening phage display libraries where, for example, the phage express scFv fragments on the surface of their coat with a large variety of complementarity determining regions (CDRs). This technique is also well known in the art. The skilled person will also be aware of techniques to improve the affinity of such scFv fragments, e.g. by rounds of mutation and selection.

### Detection of Syncytium Formation

Fusion/syncytium formation may be detected by any suitable means known in the art.

Detection may be by reporter assay such as an expression or transactivation assay. In such a system, one cell harbours a promoter-reporter nucleic acid construct, whilst the other cell expresses a transactivation element. If the contents of the two cells mix, the transactivation element is able to induce expression of the reporter, and this expression of the reporter is detected and indicates that cell fusion has taken place. Such transactivation-reporter assays are well known in the art. Examples of suitable system(s) useful in the present invention include transactivating proteins such as HIV TAT or the Tetracycline resistance operon protein which could be expressed for example in the target cell, and which could drive expression of reporters such as galactosidase, luciferase or fluorescent proteins under the control of the relevant promoter (with respect to the transactivating protein) in the test cell. Alternative configurations suitable for use in the methods of the present invention will be apparent to a person skilled in the art.

Detection may be by monitoring mixing of cell contents, or by monitoring mixing of cell membranes. These may involve monitoring of mixing of dyes loaded into one or both cells of the assay (or into their membranes), which dyes may be visually detectable or detectable by fluorescence (including detection of Fluorescent Resonance Energy Transfer or FRET, or detection by decrease of fluorescence upon syncytia formation by quenching). Detection may also be by expression of β-lactamase enzyme in the test cell and loading the target cell with a dye such as CCF-4 that on cleavage by β-lactamase yields a detectable fluorescent signal. Alternatively, the test cell may be loaded with dye and the β-lactamase expressed in the target cell.

Detection may be by visual inspection, such as by microscopy, for example phase contrast microscopy. In some cases, inspection by unaided eye may enable detection such as where syncytium formation is so advanced that the fused cell monolayer detaches from the substrate, which may be assessed by eye. Alternatively, systems measuring the size of cells, such as Coulter Counters or fluorescence activated cell analysers or sorters (FACS), measuring parameters such as forward scatter and side scatter, may be used to detect syncytium formation.

The detection techniques described herein may be selected by a person skilled in the art according to the intended application of the methods of the present invention. For example, to facilitate automation and/or to increase throughput, a detection method using reporter proteins driven by a transactivating element may be selected since such data may be easily collected by use of an array and/or a detection unit, such as a FACS machine and/or plate reader suitable for measuring absorbance, fluorescence, luminescence, or other property. Thus, in another aspect, the invention relates to assays such as high throughput assays for screening for HCV fusion/entry inhibitors and/or candidate HCV entry receptor(s).

In another example, if the methods of the present invention are applied to small scale testing in a laboratory, a microscopic detection method might be selected, since such a method requires less sophisticated equipment and may be carried out individually by the operator.

### Hepatitis C Virus

Hepatitis C Virus (HCV) is a generic term which embraces individual species and/or genotypes of HCV. 'HCV' should thus be understood to include all HCV genome types, particularly 1b and/or 1a as appropriate unless the context and/or specific statements indicate otherwise.

### Pharmaceutical Compositions

The present invention also provides a pharmaceutical composition comprising a therapeutically effective amount of the agent(s) and/or modulator(s) of the present invention and a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be administered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be administered by a number of routes.

Where the agent is to be administered mucosally through the gastrointestinal mucosa, it should be able to remain stable during transit through the gastrointestinal tract; for example, it should be resistant to proteolytic degradation, stable at acid pH and resistant to the detergent effect of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some embodiments, the agents and/or growth factors of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

If the agent/modulator is a protein, then said protein may be prepared *in situ* in the subject being treated. In this respect, nucleotide sequences encoding said protein may be delivered by use of non-viral techniques (e.g. by use of liposomes) and/or viral techniques (e.g. by use of retroviral vectors) such that the said protein is expressed from said nucleotide sequence.

In a preferred embodiment, the pharmaceutical of the present invention is administered topically. In an even more preferred embodiment, the pharmaceutical of the present invention is administered orally. Hence, preferably the pharmaceutical is in a form that is suitable for topical delivery, more preferably the pharmaceutical is in a form that is suitable for oral delivery.

### Administration

The term "administered" includes delivery by viral or non-viral techniques. Viral delivery mechanisms include but are not limited to adenoviral vectors, adeno-associated viral (AAV) vectors, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof.

The components of the present invention may be administered alone but will generally be administered as a pharmaceutical composition - e.g. when the components are in admixture with a suitable pharmaceutical excipient, diluent or carrier selected with regard to the intended route of administration and standard pharmaceutical practice.

For example, the components can be administered (e.g. orally or topically) in the form of tablets, capsules, ovules, elixirs, solutions or suspensions, which may contain flavouring or colouring agents, for immediate-, delayed-, modified-, sustained-, pulsed- or controlled-release applications.

If the pharmaceutical is a tablet, then the tablet may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The routes for administration (delivery) include, but are not limited to, one or more of: oral (e.g. as a tablet, capsule, or as an ingestable solution), topical, mucosal (e.g. as a nasal spray or aerosol for inhalation), nasal, parenteral (e.g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, vaginal, epidural, sublingual.

In a preferred aspect, the pharmaceutical composition is delivered topically. In a more preferred aspect, the pharmaceutical composition is delivered orally.

It is to be understood that not all of the components of the pharmaceutical need be administered by the same route. Likewise, if the composition comprises more than one active component, then those components may be administered by different routes.

If a component of the present invention is administered parenterally, then examples of such administration include one or more of: intravenously, intra-arterially, intraperitoneally, intrathecally, intraventricularly, intraurethrally, intrasternally, intracranially, intramuscularly or subcutaneously administering the component; and/or by using infusion techniques.

For parenteral administration, the component is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

As indicated, the component(s) of the present invention can be administered intranasally or by inhalation and is conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A™) or 1,1,1,2,3,3,3-heptafluoropropane (HFA 227EA™), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the agent and a suitable powder base such as lactose or starch.

Alternatively, the component(s) of the present invention can be administered in the form of a suppository or pessary, or it may be applied topically in the form of a gel, hydrogel, lotion, solution, cream, ointment or dusting powder. The component(s) of the present invention may also be dermally or transdermally administered, for example, by the use of a skin patch. They may also be administered by the pulmonary or rectal routes. They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the component(s) of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it can be formulated as a suitable lotion or cream suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

### Dose Levels

Typically, a physician will determine the actual dosage which will be most suitable for an individual subject. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

Depending upon the need, the agent may be administered at a dose of from 0.01 to 30 mg/kg body weight, such as from 0.1 to 10 mg/kg., more preferably from 0.1 to 1 mg/kg body weight.

### Formulation

The component(s) of the present invention may be formulated into a pharmaceutical composition, such as by mixing with one or more of a suitable carrier, diluent or excipient, by using techniques that are know in the art.

### Pharmaceutically Active Salt

The agent of the present invention may be administered as a pharmaceutically acceptable salt. Typically, a pharmaceutically acceptable salt may be readily prepared by using a desired acid or base, as appropriate. The salt may precipitate from solution and be collected by filtration or may be recovered by evaporation of the solvent.

### Treatment

It is to be appreciated that all references herein to treatment include one or more of curative, palliative and prophylactic treatment. Preferably, the term treatment includes at least curative treatment and/or prophylactic treatment.

The treatment may be of one or more of chronic/acute hepatitis, HCV infection, or related complaint.

### Therapy

The agents/modulators identified by the methods of the present invention may be used as therapeutic agents - i.e. in therapy applications. As with the term "treatment", the term "therapy" includes curative effects, alleviation effects, and prophylactic effects. The therapy may be on humans or animals.

The therapy can include the treatment of one or more of chronic/acute hepatitis, HCV infection, or related complaint.

The present invention will be described by way of example with reference to the following figures:

The figures demonstrate, *inter alia,* that cell surface expressed HCV envelope proteins promote cell fusion, as observed by syncytium formation.
Figure 1, which shows a photomicrograph of CHO+HepG2 cells, 24 hours post seeding, superconfluent monolayer.
Figure 2, which shows a photomicrograph of CHO E1E2+HepG2 cells, 24 hours post seeding, showing syncytium formation.
Figure 3, which shows a photomicrograph of CHO E1E2+HepG2 cells + soluble E2 protein, 24 hours post seeding, superconfluent monolayer.
Figure 4, which shows a photomicrograph/fluorescence photomicrograph of CHO E1E2 labelled with R-18 + Huh7 (a) and of CHO E1E2 + Huh7 labelled with R-18 (b)
Figure 5, which shows a photomicrograph/fluorescence photomicrograph of a syncytium formed by GFP-expressing Huh7 cells and CHO E1E2 cells
Figure 6, which shows the results of a reporter assay detecting syncytium formation
Figure 7, which shows the inhibition of syncytium formation by antibodies to E2: AP98. (a), and AP33 (b)
Figure 8, which shows photomicrographs showing the inhibition of syncytium formation by antibodies to LDL receptor
Figure 9, which shows photomicrographs showing the inhibition of syncytium formation by a chemical compound

### EXAMPLES

### EXAMPLE 1: ASSAY FOR SYNCYTIUM FORMATION

In this Example, a method for producing and monitoring syncytium formation between test cells and candidate cells ('target cells') is demonstrated.

Chimeric Hepatitis C Virus envelope glycoproteins having the ectodomains of the E1 and E2 HCV envelope proteins and the transmembrane and cytoplasmic domains of vesicular stomatitis virus G protein were expressed on the cell surface of Chinese Hamster Ovary cells (Takikawa *et al.* (2000) *J. Virol.* vol 74 pp 5066-5074). These test cells are referred to as 'CHO E1E2'.

The VSV-G derived domain(s), particularly the VSV-G transmembrane ('VSV-G-TM') domain, may be replaced with other(s), for example with the Sindbis TM domain, but in this example the VSV-G TM domains were used.

CHO E1E2 were maintained in DMEM, 10% Foetal Calf Serum, 2mM L-glutamine and are periodically passaged in Geneticin (500µg/ml) and Puromycin (100µg/ml). Cell surface expression of E1 and E2 envelope proteins was confirmed by immunofluoresence using antibodies to E1 and to E2. CHO cells which express the HCV virus envelope proteins E1 and E2 (CHO E1E2) at the cell surface were detached from culture flasks using either 'cell dissociation fluid' (Sigma, cat. No. C5789) according to the manufacturer's instructions, or using standard trypsinisation.

'Target' cells (HepG2 cells in this Example) were detached from plastic flasks as above.

CHO E1E2 and 'target' cells were mixed at a ration of 1:2 - 1:8 in cell culture media B (DMEM, 10% Foetal Calf Serum, 2mM L-glutamine, 1mM Sodium Pyruvate, 1% non-essential amino acids). Cell mixes were seeded in multiwell plates at densities empirically determined to give exact confluency on seeding; 6 well plates were seeded at 200 x 10⁴ cells/well, 96 well plates were seeded at 8 x 10⁴ cells/well.

Controls of nontransformed CHO cells were also mixed with 'target' cells and seeded as above.

Cells were incubated at 37°C, 5% CO₂ for 20-36 hours. Cells were inspected for syncytium formation at regular intervals from 16 hours onwards.

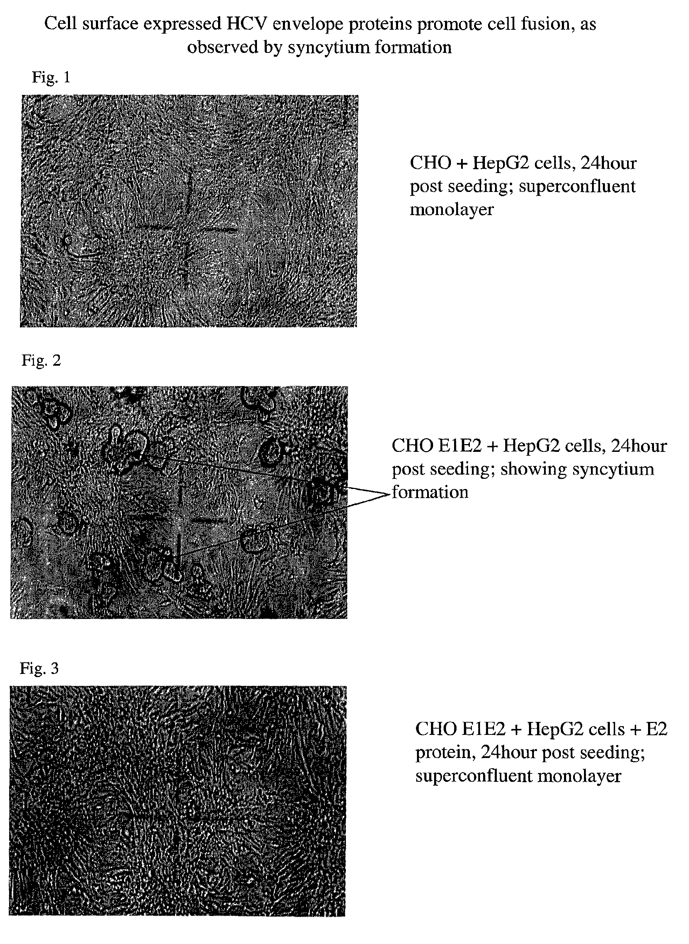

From approximately 20 hours post-seeding, syncytium formation across the cell monolayer was observed. In many cases this progressed to complete detachment of portions of the monolayer (Fig.2).

CHO cells which do not express HCV E1 and E2 proteins did not exhibit detectable syncytium formation with 'permissive' cells under these experimental conditions (Fig.1).

Thus, the assay of syncytium formation according to the present invention is demonstrated.

### EXAMPLE 2: DETERMINATION OF PERMISSIVE / NONPERMISSIVE CELL TYPES

A permissive cell type according to this Example is one which will undergo fusion with the CHO E1E2 test cells under the conditions of the assay as set out in Example 1. The cell type for which permissiveness is to be determined is the target cell.

Huh7 cells and HepG2 cells (from ATCC) were maintained in cell culture medium B (DMEM, 10% Foetal Calf Serum, 2mM L-glutamine, 1mM Sodium Pyruvate, 1% non-essential amino acids).

The assay protocol of Example 1 was used to evaluate a range of mammalian cell types for CHO E1E2 induced syncytium formation i.e. permissiveness.

Cell lines identified to be permissive include HepG2 (human hepatoma) and Huh7 (human hepatoma). Without wishing to be bound by theory, hepatocytes are envisaged to be the primary source of HCV replication, although not all tissue culture adapted hepatocytes are anticipated to be permissive for HCV fusion (C.H. Hagedorn and C. M. Rice, The Hepatitis C Viruses, Springer, p 266).

Cell lines identified to be non-permissive include: Chang, Hela (human cervical carcinoma), Hek293 (Human Embryonic Kidney), Cos-7 (African Green Monkey kidney cells, SV40-transformed) and CHO (Chinese Hamster Ovary) cells.

The syncytium formation assays/methods of the present invention are based on real biological events (i.e. syncytium formation) as distinct from those of a 'fusion' assay report (Takikawa *et al.* (2000) *J. Virol.* vol 74 pp 5066-5074) which relied on indirect reporters in the absence of syncytium formation. The prior art reporter protein protocol showed highest fusion activity for a HepG2 cell line (7x signal:noise), whereas, both Huh7 and Hela cells 'showed little fusion activity' (3x signal:noise). In contrast, using the syncytium formation protocols of the present invention we have found that both HepG2 and Huh7 cell lines show equal permissiveness for fusion, whereas, Hela cells were entirely non-permissive.

### EXAMPLE 3: E2 DEPENDENCY OF SYNCYTIUM FORMATION

The syncytium formation assay of Example 1 was conducted in the presence and absence of soluble E2 protein in order to demonstrate the effect(s) of E2 on syncytium formation. Syncytium formation in this Example is treated as an indicator of the underlying mechanism of viral fusion/entry.

Test cells and target cells were prepared as in Example 1. Recombinant E2 protein (from E2 protein stock solution: 0.2mg/ml from Austral cat. no. HCA-090-2) was added to a final concentration of 800nM immediately subsequent to cell seeding.

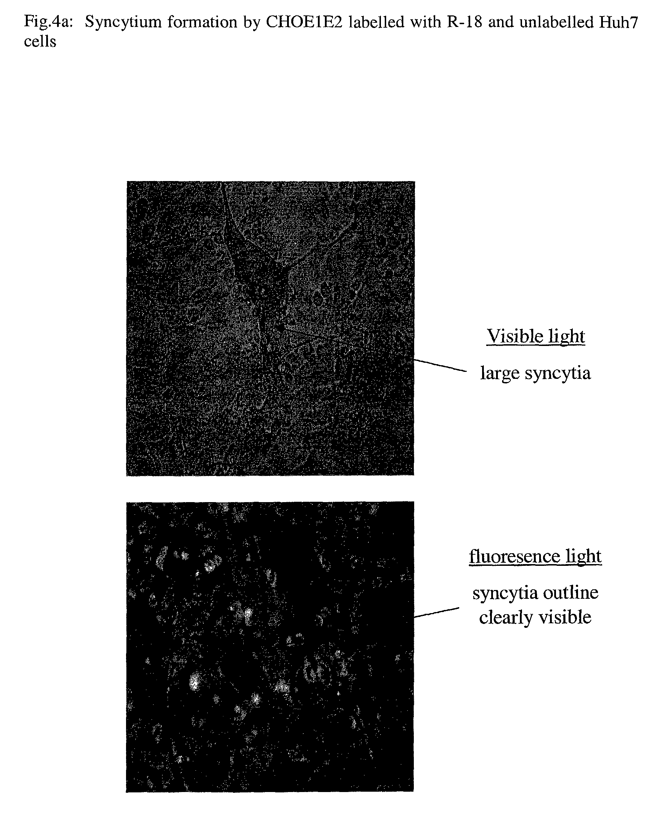

Syncytium formation was completely inhibited in the presence of 800nM recombinant E2 protein (Fig.3). A dose dependent inhibitory effect was observed at lower E2 concentrations. There was no visible evidence of cytotoxicity associated with E2 protein.

Thus it is demonstrated that recombinant E2 protein is a potent inhibitor of syncytium formation.

### EXAMPLE 4: E1 DEPENDENCY OF SYNCYTIUM FORMATION

Studies using E1 antisera and peptides show E1 dependency of syncytium formation. The syncytium formation assay of Example 1 was conducted in the presence and absence of E1 antisera and/or peptides in order to demonstrate the effect(s) of E1 on syncytium formation. Syncytium formation is treated as an indicator of the underlying mechanism of viral fusion/entry.

Test cells and target cells are prepared as in Example 1. E1 antisera and/or peptides are added to the media immediately subsequent to cell seeding. Syncytium formation is modulated/inhibited in the presence of E1 antisera and/or peptides.

Thus it is demonstrated that E1 antisera and/or peptides are potent inhibitor(s) of syncytium formation.

### EXAMPLE 5: DETECTION OF SYNCYTIUM FORMATION BY DETECTING MEMBRANE FUSION

### a) Proof of Principle

CHO E1E2 were maintained in Dulbeccos Modified Eagles Medium (DMEM), 10% Foetal Calf Serum (FCS), 2mM L-glutamine and occasional passage in Geneticin (500µg/ml) and Puromycin (100µg/ml). Huh7 were maintained in DMEM, 10% FCS, 2mM L-glutamine, 1mM Sodium Pyruvate, 1% non-essential amino acids. The fluorescent lipid probe R-18 (Molecular Probes cat no. O-246) was made to 10mg/ml in ethanol. This R-18 stock solution was added to culture medium to a final concentration of 3µg/ml for CHO E1E2 cultures or 1µg/ml for Huh7 cultures. The cells were maintained in their respective R-18-containing media for 16 hours at 37°C, 4% CO₂.

The CHO E1E2 and Huh7 cells were detached from the plastic flasks using either trypsin or 'cell dissociation fluid'. CHO E1E2 and Huh7 cells were mixed at a ratio of 1:2 in Huh7 cell culture medium. The cell mixes were seeded in 96 well plates at a density of 8 x10⁴ cells/well. The cells were then incubated at 37°C, 4% CO₂ for 24 hours. The cell monolayers were examined by light microscopy and by fluorescence microscopy (using filters for excitation at 530nm and emission at 590nm).

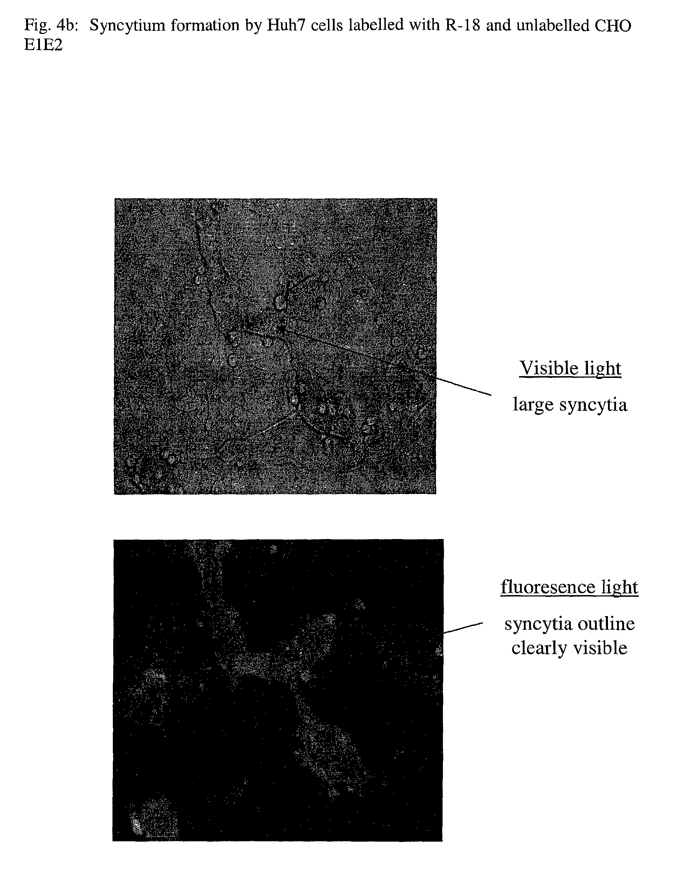

The results are shown in Figure 4. Fluorescence was observed in syncytium structures. Similar results were obtained when the assay was carried out with R-18 labelled CHO E1E2 cells and unlabelled Huh7 cells (Figure 4a), as when R-18 labelled Huh7 cells and unlabelled CHO E1E2 cells were used (Figure 4b). This shows that true mixing of the membranes from the two cell types occurs.

This assay can be adapted to a format suitable for high throughput screening.

### b) High throughput assay format (1)

CHO E1E2 cells and Huh7 cells are grown as described above. CHO E1E2 cells are then labelled with R-18 as described above; Huh7 cells are labelled with F-18 (Molecular Probes, Cat No O-3852), as per manufacturer's instructions. After incubation for 16 hours at 37°C, 4% CO₂, the cells are stripped from the plastic flasks and mixed as described above, and seeded in multi-well dishes (e.g. 96 well plates or 384 well plates) at the densities as described above to give confluency. Test compounds are added to most wells, whereas only the corresponding amount of the diluent used for the test compounds is added to control wells.

After incubation for about 20 hours, syncytium formation is detected by an increase in fluorescence at the emission wavelength of F-18, which occurs through fluorescence resonance energy transfer (FRET) between the two dyes when they are brought into close proximity with each other. When test compounds inhibit syncytium formation, the wells treated with these compounds will not show the fluorescence increase as observed in the control wells, where syncytium formation occurs.

The skilled person will appreciate that the dyes used in the above assay are only an example, and that this method can be performed with other fluorophore pairs known in the art.

### (c) High throughput assay format (2)

CHO E1E2 cells and Huh7 cells are grown as described above. CHO E1E2 cells are then labelled with R-18 as described above, but using a final concentration of 10µg/ml of R-18. After incubation for 16 hours at 37°C, 4% CO₂, the R-18 labelled CHO E1E2 cells and the unlabelled Huh7 cells are stripped from the plastic flasks and mixed as described above at a ratio of CHO E1E2 to Huh7 of about 1:20, and seeded in multi-well dishes (e.g. 96 well plates or 384 well plates) at the approporate density to give confluency. Test compounds are added to most wells, whereas only the corresponding volume of the diluent used for the test compounds is added to control wells.

Syncytium formation in control wells is detected by an increase in fluorescence at the R-18 emission wavelength through effective dilution of R-18 resulting in loss of self-quenching, whereas in wells where the test compound has inhibited syncytium formation the fluorescence is low as the R-18 will not be diluted, and therefore its fluorescence remains quenched.

The skilled person will appreciate that R-18 is just one example of a fluorophore that can be used in this method, and that other fluorophores with appropriate properties can be used instead.

### EXAMPLE 6: DETECTION OF SYNCYTIUM FORMATION BY DETECTING MIXING OF CYTOPLASM

### a) Proof of Principle

CHO E1E2 were maintained in Dulbeccos Modified Eagles Medium (DMEM), 10% Foetal Calf Serum (FCS), 2mM L-glutamine and occasional passage in Geneticin (500µg/ml) and Puromycin (100µg/ml). Huh7 were maintained in DMEM, 10% FCS, 2mM L-glutamine, 1mM Sodium Pyruvate, 1% non-essential amino acids.

Huh7 cells were transiently transfected with a plasmid for expression of Green Fluorescent Protein (GFP) in mammalian cells (e.g. pHygEGFP, Clontech, Cat No 6014-1). Typical transfections use lipid-based transfection techniques, e.g. Effectene (Qiagen, Cat No 301425), used as per manufacturer's instructions.

CHO E1E2 cells and transfected Huh7 cells were stripped from plastic flasks using standard techniques (trypsinisation or cell dissociation fluid from Sigma). CHO E1E2 cells are mixed with transfected Huh7 cells at a ratio of 1:2 in Huh7 cell culture medium. Cell mixes were seeded in 96 well plates at 8x10⁴ cells/well. Cells were incubated at 37°C, 4% CO₂ for 24 hours. Cell monolayers were examined by light microscopy and fluorescence microscopy (using filters for excitation at 490nm, and emission at 530nm).

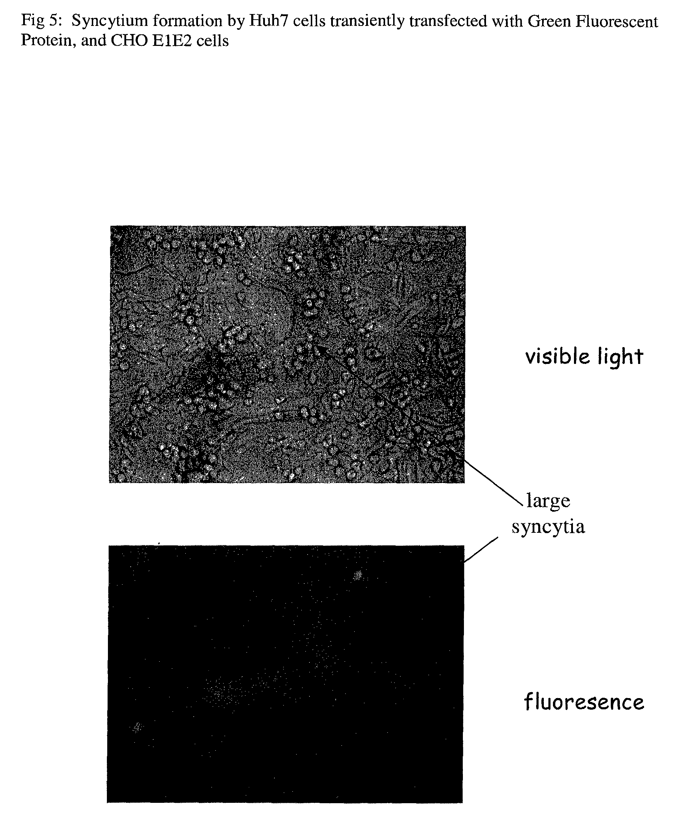

The results are shown in Figure 5. GFP-labelled cytoplasm was demonstrated to diffuse freely throughout the syncytium structure.

### b) Assay formats suitable for high throughput screening

Cytoplasmic mixing in syncytia can be used to set up screens, e.g. to identify compounds that will inhibit syncytium formation, and therefore can be useful for inhibiting HCV entry into cells. One example is an assay based on fluorescence resonance energy transfer (FRET). The cells are grown as described in previous examples. The CHO E1E2 cells are loaded with a probe tagged with rhodamine, the Huh7 cells are loaded with a probe tagged with fluorescein. The cells are mixed, seeded at the appropriate densities in multi-well plates as described above (e.g. 96 well plates or 384 well plates), and test compounds are added to most wells, whereas the same volume of the diluent without compound is added to control wells (for the preparation of the compounds, see Example 8). After incubation as described above, syncytium formation is detected by the increase in fluorescence through FRET where the two fluorophores have come into close proximity by mixing of the cytoplasm of the two cell types after syncytium formation, whereas in the samples where the test compound has inhibited syncytium formation, the increase in fluorescence is not observed.

The skilled person will appreciate that other appropriate fluorophore pairs can be used in such an assay system.

### EXAMPLE 7: REPORTER ASSAYS TO DETECT SYNCYTIUM FORMATION

When cells expressing a transactivator are mixed with cells expressing a reporter protein under the control of a transactivator-dependent promoter, then on cell-cell fusion the transactivator will turn on the production of the reporter protein, and the increase in the reporter protein can be easily detected. Examples of such systems are
1) the HIV tat peptide driving a reporter protein under the control of the HIV LTR promoter. The LTR promoter may be composed of the complete LTR sequence or truncated regions of the LTR sequence
2) the Tetracycline regulatory protein driving a reporter protein under the control of the Tetracycline response element

Examples for such reporter proteins include β galactosidase, luciferase, β lactamase, or fluorescent proteins such as GFP. Such reporter-based assay systems can be used for the detection of syncytium formation according to the invention in several formats; e.g.
- CHOE1E2 transactivator + hepatocyte reporter
- CHOE1E2 reporter + hepatocyte transactivator
- CHOE1E2 + hepatocyte reporter + hepatocyte transactivator
These assays can be used in high throughput screening for modulators of HCV entry.

Using standard techniques well established in the art, Huh7 cells were engineered to stably express the HIV tat peptide (using the Tat coding sequence from the HIV Lai isolate, Genbank accession number K02013, nucleotides 5412-5626, cloned into pcDNA3.1+ (Invitrogen Cat No v790-20)), resulting in a cell line called Huh7-pTAT. Similarly, Huh7 cells were engineered to stably contain an HIV LTR-luciferase construct (using the LTR from the HIV Lai isolate, Genbank accession number K02013, nucleotides 1-791, cloned into the promoterless luciferase vector pGL2 basic plasmid, available from Promega, Cat No E1641, Genbank accession number X65323), resulting in a cell line called Huh7-pLTR-luciferase.

CHO E1E2 were maintained in DMEM, 10% FCS, 2mM L-glutamine and occasional passage in Geneticin (500µg/ml) and Puromycin (100µg/ml). Huh7-pTAT were maintained in DMEM, 10% FCS, 2mM L-glutamine, 1mM Sodium Pyruvate, 1% non-essential amino acids, 800µg/ml Geneticin. Huh7-pLTR-luciferase were maintained in DMEM, 10% FCS, 2mM L-glutamine, 1mM Sodium Pyruvate, 1% non-essential amino acids, 800µg/ml Geneticin.

CHO E1E2, Huh7-pTAT and Huh7-pLTR-luciferase were stripped from plastic flasks using standard techniques, either with trypsin or 'cell dissociation fluid' (Sigma). The ratio of Huh7-pTAT to Huh7-pLTR-luciferase was 1:1. The CHO E1E2 and Huh7-pTAT and Huh7-pLTR-luciferase lines were mixed at a ratio of 1:2, 1:6 or 1:10 in Huh 7 cell culture media, and seeded in 96 well plates at 8 x10⁴ cells/well. For example for a CHO E1E2:Huh7 ratio of 1:2, cells would be seeded as CHO E1E2 at 2.7 x10⁴cells/well + Huh7-pTAT at 2.7 x10⁴cells/well + Huh7pLTR-luciferase at 2.7 x10⁴cells/well.

Controls of nontransformed CHO cells were also mixed with Huh7-pTAT and Huh7-pLTR-luciferase, and were seeded as described above. Cells were incubated at 37°C, 4% CO₂ for 24 hours. The luciferase activity of cell lysates was measured by standard techniques e.g. Promega Luciferase Assay System, Cat. E1501. The luciferase counts were measured in plate readers such as the Wallac ' Victor multi label counter'.

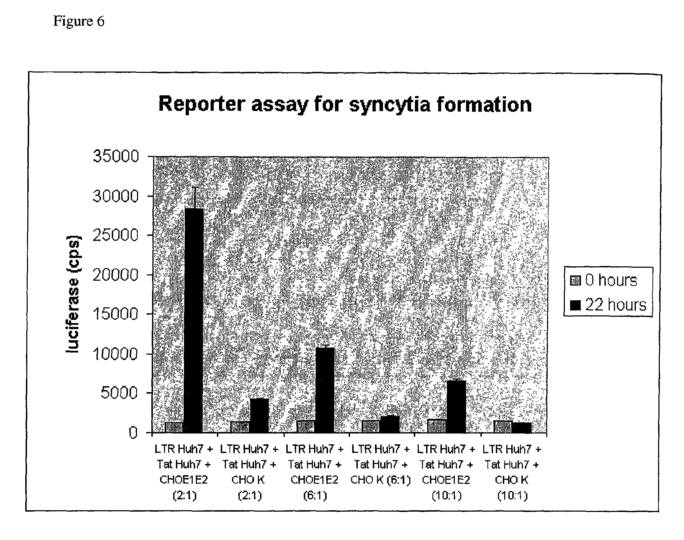

The results are shown in Figure 6, and Table 1. A CHO E1E2-dependent reporter signal that ranged from 5-7 fold above background was obtained in this study.

A reporter system for detecting syncytium formation has clearly been demonstrated. A reporter system as above could be used to measure cell-cell fusion events before syncytia formation can be detected visually, it therefore represents potentially a more sensitive assay format than visual detection.

**Table 1:**

| Reporter assay for detection of syncytium formation. All data are given as luciferase activity (cps), with 4 measurements per sample (n=4); the standard deviation is indicated. | | |
|---|---|---|
| | 0 hours | 22 hours |
| CHO E1E2 + Huh7-pTAT + Huh7-pLTR-luciferase (1:2) | 1284 +/- 45.3 | 28327 +/- 2727.5 |
| CHO + Huh7-pTAT + Huh7-pLTR-luciferase (1:2) | 1383 +/- 43.9 | 4215 +/- 177.8 |
| CHO E1E2 + Huh7-pTAT + Huh7-pLTR-luciferase (1:6) | 1581 +/- 93.5 | 10737 +/- 381.7 |
| CHO + Huh7-pTAT + Huh7-pLTR-luciferase (1:6) | 1543 +/- 41.6 | 2048 +/- 100.7 |
| CHO E1E2 + Huh7-pTAT + Huh7-pLTR-luciferase (1:10) | 1671 +/- 63.5 | 6649 +/- 204.1 |
| CHO + Huh7-pTAT + Huh7-pLTR-luciferase (1:10) | 1500 +/- 52.6 | 1280 +/- 22.9 |

### EXAMPLE 8: REPORTER ASSAY FOR SCREENING COMPOUNDS FOR INHIBITORS OF SYNCYTIUM FORMATION

The cell lines were maintained as described above.

Compounds were made to a 4mM stock in 100% DMSO. The compounds were diluted in Huh7 cell culture medium to 10µM. The compounds were added to 96 well plates to give a final assay concentration of 1µM.

A bulk mix of CHO E1E2 and the Huh7-pTAT and Huh7-pLTR-luciferase lines were prepared in Huh 7 cell culture media as described in Example 7. The ratio of Huh7-pTAT to Huh7-pLTR-luciferase was 1:1. The CHO E1E2 and Huh7 lines were mixed at a ratio of 1:6, and seeded in a 96 well plate to give a total of 8 x10⁴ cells/well (i.e. CHO E1E2 at 1.14 x10⁴cells/well, Huh7-pTAT at 3.4 x10⁴cells/well, Huh7-pLTR-luciferase at 3.4 x10⁴cells/well. The cells were added to the 96 well plate already prepared with compound. Cells were incubated at 37°C, 4% CO₂ for 24 hours.

The luciferase activity of cell lysates was measured by standard techniques e.g. Promega Luciferase Assay System, Cat. no E1501. The luciferase counts were measured in plate readers such as the Wallac ' Victor multi label counter'.

Compounds that may inhibit syncytium formation are identified by low luciferase activity being detected in the corresponding well. Any compounds showing inhibition of reporter signal are repeated and profiled over a 3 log range of concentrations to give a final assay concentration of 30 - 0.03µM.

This example demonstrates that the reporter assay can be used in high throughput format to identify inhibitors of syncytia formation.

### EXAMPLE 9: INHIBITION OF SYNCYTIUM FORMATION BY ANTIBODIES TO E2

The assay as described in Example 8 was carried out, to test whether antibodies to E2 would inhibit syncytium formation. Anti-E2 monoclonal antibodies AP98 and AP33 (obtained from Dr. Arvind Patel, see Owsianka *et al,* Journal of General Virology (2001), vol 82, 1877-1883; the AP33 epitope consists of amino acids 412-423 of the HCV polyprotein; the AP98 epitope consists of amino acids 644-651 of the HCV polyprotein). They were serially diluted in Huh7 cell culture medium and added to a 96 well plate to give final dilutions of 1/10, 1/20, 1/40, and 1/80. The cell mixture was added to the plate as described above, and after incubation for 28 hours, the luciferase assay was performed.

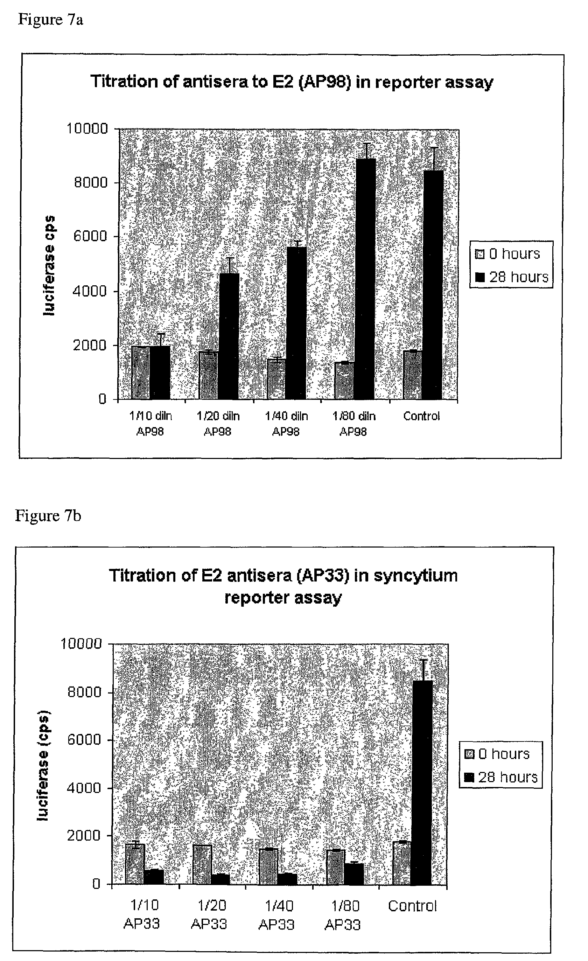

The results are shown iwn Figure 7. Figure 7a shows the result for AP98, Figure 7b with AP33. The results for AP98 are also shown in Table 2:

**Table 2:**

| Inhibition of syncytium formation with anti-E2 antiserum AP98. The results shown are luciferase activity (cps) per well. The experiment was done with n=4, the standard deviation is given. | | |
|---|---|---|
| Dilution / Time | 0 hours | 28 hours |
| Control | 1791 +/- 47.9 | 8464 +/- 889.7 |
| 1/10 | 1939 +/- 27.5 | 1959 +/- 467.0 |
| 1/20 | 1750 +/- 70.2 | 4658 +/- 581.8 |
| 1/40 | 1477 +/- 95.0 | 5630 +/- 242.4 |
| 1/80 | 1365 +/- 40.2 | 8923 +/- 582.5 |

The results show that the assay can identify inhibitors of syncytium formation. It also confirms that syncytium formation is dependent on E2.

The assay is also used to evaluate peptide mimics of the E1 and E2 proteins for their ability to inhibit syncytium formation. Sequential peptides spanning the E1 and E2 region are synthesised, peptides are 25mers with an overlap of 8 amino acids. Peptides are made to 2 mg/ml in dH₂0 and then diluted and titrated in Huh7 culture media and added to a 96 well plate to give a final concentration of 200-0.2µg/ml.

The assay is performed as described above. Decreased luciferase activity for certain peptides indicates that the peptide can act as an inhibitor of syncytium formation, and confirms that syncytium formation is dependent on E1 and/or E2.

### EXAMPLE 10: INHIBITION OF SYNCYTIUM FORMATION BY ANTISERUM TO AN ENTRY FACTOR

The LDL receptor has been postulated to be involved in HCV entry into hepatocytes (Agnello *et al,* (1999) Proc. Natl. Acad. Sci USA. vol 96, 12766-12771). Syncytium formation as described herein can be used to test whether a cell surface receptor is involved in HCV entry, for example by testing whether an antibody to the receptor can block syncytium formation as described herein.

A monoclonal antibody to human LDL receptor (available from Research Diagnostics Inc., cat.no RDI-PRO61087) was serially diluted in Huh7 cell culture medium and added to a 96 well plate to give final concentrations of 5 µg/ml, 1.7 µg/ml, 0.5 µg/ml, and 0.17 µg/ml.

A bulk mixture of CHO E1E2 and Huh7 cells was prepared in Huh7 cell culture medium at a ratio of 1:2, and a total of 8 x10⁴ cells/well were seeded in a 96 well plate (i.e. CHO E1E2 at 2.8 x10⁴cells/well, HepG2 at 5.2 x10⁴cells/well). The cells were added to a 96 well plate already prepared with anti-LDL receptor antibody. The 96 well plates were then incubated at 37°C, 4% CO₂ for 24 hours. Cells were visually monitored for both syncytia formation (and inhibition thereof) and for evidence of cytotoxicity.

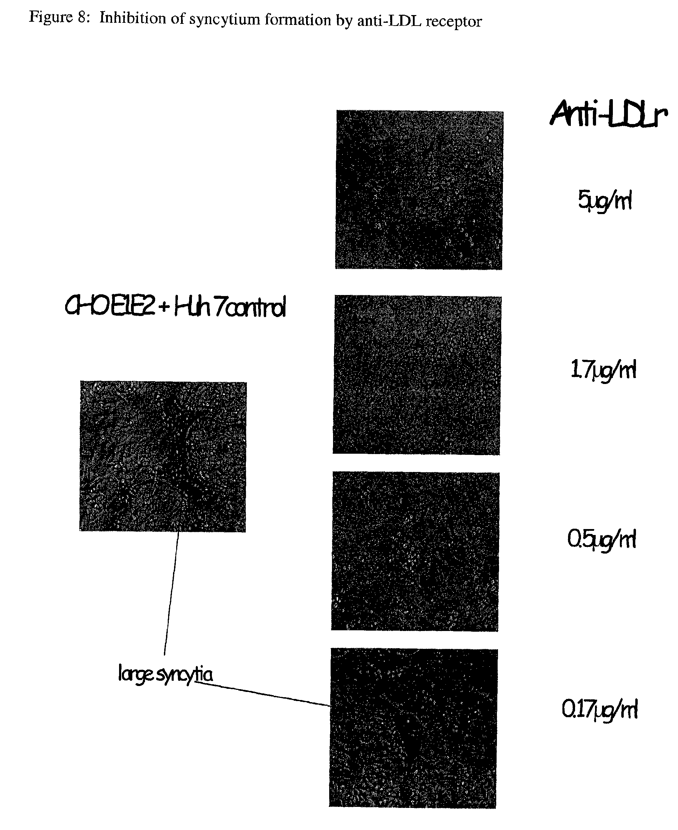

The results are shown in Figure 8. Syncytia were still formed at 0.17 µg/ml anti-LDL receptor antibody; at the higher concentrations of antibody, no syncytium formation could be detected.

The results show that antibodies or compounds binding to HCV receptors inhibit syncytium formation, which is a model for HCV entry into cells.

The example also shows that the assay described herein can be used to identify HCV entry factors. Collections of available antisera may be screened in this assay format to identify host cell molecules important in HCV entry. Antisera raised to liver cell membranes would be particularly suitable for identification of candidate HCV entry factors.

### EXAMPLE 11: INHIBITION OF SYNCYTIUM FORMATION BY A COMPOUND

CHOE1E2 were maintained in DMEM, 10% FCS, 2mM L-glutamine and occasional passage in Geneticin (500µg/ml) and Puromycin (100µg/ml). HepG2 were maintained in DMEM, 10% FCS, 2mM L-glutamine, 1mM Sodium Pyruvate, 1% non-essential amino acids. CHOE1E2 and HepG2 were detached from plastic flasks using either trypsin or 'cell dissociation fluid'.

Compounds were made to a 4mM stock in 100% DMSO. Compounds were then diluted in HepG2 cell culture medium to 10µM. The compounds were added to 96 well plates to give a final assay concentration of 1µM. Any compounds showing inhibition of syncytia formation were then profiled over a 3 log range of concentrations to give a final assay concentration of 30 - 0.03µM.

A bulk mix of CHOE1E2 and HepG2 was prepared in HepG2 cell culture medium at a ratio of 1:2 and a total of 8 x10⁴ cells/well in a 96 well plate, i.e. CHOE1E2 at 2.8 x10⁴cells/well, HepG2 at 5.2 x10⁴cells/well. The cells were seeded to a 96 well plate already containing the compound solution, prepared as described above. The plates were incubated at 37°C, 4% CO₂ for 24 hours. The plates were then visually inspected for both inhibition of syncytia formation and evidence of cytotoxicity.

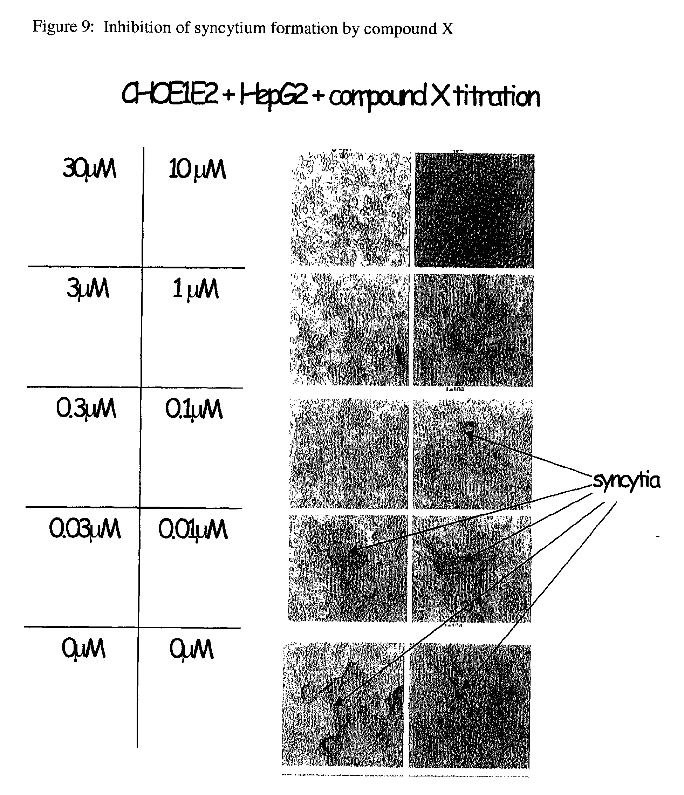

The results with one example of an inhibiting compound (compound X) are shown in Figure 9. Inhibition of formation of syncytia was observed with an IC₅₀ of about 0.5µM (by visual inspection), whereas overt cytotoxicity was observed at a concentration of ≥ 10µM (by visual inspection). A dose response titration for inhibition of syncytia formation was observed. Inhibition of syncytia formation could be clearly distinguished from overt cytotoxic effects.

The example clearly demonstrates that chemical compounds inhibiting syncytium formation can be found. In this case, visual inspection was used, which is fairly low throughput. Other assay formats have been exemplified which will be suitable for high throughput screening for inhibitors of syncytium formation. As syncytium formation represents a model system for HCV entry, the inhibitors found in such assays are believed to be HCV entry inhibitors.

### REFERENCES:

### Fields Virology, 3^{rd} edition, Lippincott-Raven

Hong *et al*., New reporter cell lines to study macrophage-tropic HIV envelope protein-mediated cell-cell fusion. *AIDS Res Hum Retroviruses* (1999) Dec 10;15(18):1667-72 Danieli T *et al*., Membrane fusion mediated by the influenza virus hemagglutinin requires the concerted action of at least three hemagglutinin trimers. *J Cell Biol* (1996) May;133(3):559-69

## Claims

1. A method for identifying a candidate HCV entry factor comprising
(i) expressing a target polypeptide on the surface of a target cell
(ii) providing a test cell
(iii) mixing said test and target cells
(iv) incubating the cell mixture of (iii)
(v) assaying for syncytium formation
wherein the detection of syncytium formation indicates that the target polypeptide is a candidate HCV entry factor.

2. A method for identifying a permissive cell comprising
(i) providing a test cell
(ii) providing a candidate cell
(iii) mixing said test and candidate cells
(iv) incubating the cell mixture of (iii)
(v) assaying for syncytium formation,
wherein the detection of syncytium formation indicates that the candidate cell is a permissive cell.

3. A method for identifying a candidate modulator of HCV entry comprising
(i) providing test cells
(ii) providing target cells
(iii) mixing said test and target cells
(iv) dividing the mixture of (iii) into at least two samples
(v) introducing a test molecule into the first sample of (iv)
(vi) incubating the first and second samples
(vii) assaying the samples for syncytium formation
wherein a difference in the level of syncytium formation between the first and second samples indicates that the test molecule a candidate modulator of HCV entry.

4. A method for monitoring cell fusion, said method comprising
(i) providing a test cell
(ii) providing a target cell
(iii) mixing said test and target cells
(iv) incubating the cell mixture of (iii)
(v) assaying for syncytium formation, and
(vi) inferring the level of cell fusion from the level of syncytium formation.

5. A method according to any one of claims 1 to 4 wherein the test cell comprises one or more Hepatitis C Virus (HCV) envelope protein(s).

6. A method according to claim 5 wherein the test cell comprises Hepatitis C Virus (HCV) E1 and E2 envelope proteins.

7. A method according to any preceding claim wherein the test cell is a Chinese Hamster Ovary (CHO) derived cell.

8. A method according to any preceding claim wherein the test cells and target cells are mixed in suspension.

9. A method according to any preceding claim wherein the cells are incubated in the presence of a solid tissue culture substrate.

10. A method according to any preceding claim wherein the test cells and/or the target cells do not adhere to a solid tissue culture substrate during incubation.

11. A method according to any one of claims 1 to 9 wherein cells are incubated at a density giving substantial confluency.

12. A method according to any preceding claim wherein test cells are present at a lower concentration than target cells.

13. A method according to any preceding claim wherein test cells are present at a higher concentration than target cells.

14. A method according to claim 12 or claim 13 wherein the test cell to target cell ratio is in the range of 1:20 to 20:1.

15. A method according to claim 12 or claim 14 wherein the test cell to target cell ratio is in the range of 1:2 to 1:8.

16. A method according to any preceding claim wherein cells are incubated for at least about 4 hours.

17. A method according to any preceding claim wherein cells are incubated for at least about 20 hours.

18. A modulator of fusion identified by a method according to any preceding claim.

19. Use of E2 or parts thereof as a modulator of HCV entry.

20. Use of E2 or parts thereof in the manufacture of a medicament for treatment of HCV infection.

21. Use of E1 or parts thereof as a modulator of HCV entry.

22. Use of E1 or parts thereof in the manufacture of a medicament for treatment of HCV infection.

23. Use of an antibody to E1, E2, or an HCV entry factor as identified in claim 1 as a modulator of HCV entry.

24. Use of an antibody to E1, E2 or an HCV entry factor as identified in claim 1 in the manufacture of a medicament for treatment of HCV infection.

25. Use of claim 23 or claim 24 wherein the HCV entry factor is LDL receptor.
